Europäisches Patentamt

European Patent Office

Office européen des brevets

(19)

(11) **EP 0 556 084 B1**

(12) **FASCICULE DE BREVET EUROPEEN**

(45) Date de publication et mention
de la délivrance du brevet:
**03.04.1996 Bulletin 1996/14**

(51) Int. Cl.[6]: **C07C 51/367**, C07C 59/52

(21) Numéro de dépôt: **93400211.4**

(22) Date de dépôt: **28.01.1993**

(54) **Procédé d'obtention de l'acide orthohydroxymandélique et de ses sels**

Verfahren zur Darstellung von o-Hydroxymandelsäure und ihren Salzen

Method of preparation of orthohydroxy mandelic acid and its salts

(84) Etats contractants désignés:
**AT BE CH DE ES GB IT LI NL SE**

(30) Priorité: **12.02.1992 FR 9201565**

(43) Date de publication de la demande:
**18.08.1993 Bulletin 1993/33**

(73) Titulaire: **SOCIETE FRANCAISE HOECHST
Société anonyme dite:
F-92800 Puteaux (FR)**

(72) Inventeurs:
• **Schouteeten, Alain
F-95460 Ezanville (FR)**
• **Christidis, Yani
F-75019 Paris (FR)**

(74) Mandataire: **Rinuy, Santarelli
14, avenue de la Grande Armée
F-75017 Paris (FR)**

(56) Documents cités:
**FR-A- 2 638 740**

• **RECUEIL DES TRAVAUX CHIMIQUES DES PAYS-
BAS, vol. 107, Mars 1988, La Haye, NL, pages 242
- 247, A. J. HOEFNAGEL et al, "Metal ion catalysis
in the hydroxyalkylation of phenol with glyoxylic
acid"**

**Description**

La présente invention concerne un procédé d'obtention de l'acide orthohydroxymandélique et de ses sels.

L'acide orthohydroxymandélique est largement décrit dans la littérature, et c'est un intermédiaire précieux pour accéder à l'acide orthohydroxyphénylacétique couramment utilisé aujourd'hui dans l'obtention de molécules présentant d'intéressantes propriétés thérapeutiques ou phytosanitaires.

L'acide orthohydroxymandélique n'est pas connu à l'état cristallisé pur. Il a été isolé sous forme d'huile et il a été caractérisé notamment par son dérivé diacétylé (R.HOWE et al, J.Chem. Soc., 1967, 2510-2514).

Il est connu que l'acide glyoxylique se condense avec le phénol en milieu aqueux alcalin pour conduire à un mélange d'acides ortho ou parahydroxymandéliques, ainsi qu'aux acides hydroxybenzènediglycoliques substitués en 2,4 et en 2,6.

Il est également connu qu'en milieu aqueux alcalin, l'acide glyoxylique se dismute selon Cannizzaro en acides oxalique et glycolique. Shouteeten et Christidis ont montré qu'en faisant réagir de l'acide glyoxylique avec du phénol pendant quelques minutes, à température élevée, dans un milieu aqueux alcalin, il était possible de minimiser la réaction de Cannizzaro et d'exalter la réaction de condensation et ainsi, de pouvoir atteindre un rendement de 70 à 85 % en acide parahydroxymandélique (brevet français N° 2440350).

Par ailleurs, on sait préparer de l'acide parahydroxymandélique avec une bonne sélectivité, en milieu quasi anhydre, en faisant réagir de l'acide glyoxylique avec du phénol, en présence d'un excès d'amine tertiaire convenable tel que la tributylamine (brevet français N° 2638740).

Récemment, A.J Hoefnagel et al, Rec.Trav.Chem., 107, 242-7 (1988), ont montré que la condensation de l'acide glyoxylique avec du phénol pouvait être catalysée par certains ions métalliques et, qu'en opérant en milieu aqueux dilué, à pH = 5, à 100°C, en présence de cations métalliques trivalents tels que l'aluminium, le chrome, le fer, il était possible d'obtenir des sélectivités élevées en position ortho.

Toutefois, en raison d'une part, d'une dilution importante qui fournit une faible productivité et d'autre part, de l'obtention d'un taux élevé en produits disubstitués qui conduit à des mélanges complexes dont il est difficile ensuite d'isoler l'acide orthohydroxymandélique cherché, ce procédé n'est pas industriellement économique.

Afin d'obvier à ces inconvénients, la demanderesse a découvert avec étonnement un procédé d'obtention de l'acide orthohydroxymandélique, à partir de l'acide glyoxylique et du phénol, conduisant simultanément à une sélectivité, un rendement et une productivité élevés.

Selon l'invention, l'acide orthohydroxymandélique, ainsi que ses sels, est préparé par un procédé caractérisé en ce que l'on fait réagir de l'acide glyoxylique avec du phénol, en présence d'une amine tertiaire avec un rapport molaire amine tertiaire sur acide glyoxylique compris entre 0,8 et 1,2 et de quantités catalytiques de cations métalliques trivalents, pour obtenir l'acide orthohydroxymandélique attendu que l'on isole ou, si désiré, salifie.

L'amine tertiaire est choisie parmi les amines tertiaires, de préférence liquides ou gazeuses à la température ambiante, telles que les amines, ou leur mélange en proportions variables, de formule générale (I) $N(R_1) (R_2) (R_3)$ dans laquelle $R_1$, $R_2$, et $R_3$, identiques ou différents, représentent un radical alcoyle linéaire ou ramifié en $C_1$-$C_{10}$ ou un radical alcanol en $C_2$. A titre d'amines tertiaires, on peut citer par exemple, la triméthylamine, la triéthylamine, la tributylamine, la triéthanolamine, les mélanges d'amines tertiaires en $C_8$-$C_{10}$ commercialisés par la demanderesse sous la désignation de HOSTAREX [R] A 327. Préférentiellement, l'amine de formule générale (I) est la tributylamine.

Des cations métalliques trivalents convenables comprennent les cations aluminium (III), chrome (III), fer (III), gallium (III), indium (III), thallium (III), ruthénium (III), scandium (III), mais de préférence, les cations chrome (III), fer (III), aluminium (III) et tout particulièrement ce dernier.

Dans des conditions avantageuses de mise en oeuvre de l'invention, le procédé ci-dessus décrit est réalisé de la manière suivante :

- à une température supérieure ou égale à 50°C, avantageusement à une température supérieure à 100°C,
- à la pression atmosphérique,
- en présence de tributylamine,
- avec un rapport molaire phénol sur acide glyoxylique supérieur à 1, avantageusement supérieur à 4,
- dans un milieu quasi anhydre,
- avec un rapport molaire amine tertiaire sur acide glyoxylique compris entre 0,9 et 1,1,
- en présence de cations aluminium (III), chrome (III) ou fer (III), préférentiellement en présence de cations d'aluminium (III),
- avec un rapport molaire cations métalliques sur acide glyoxylique compris entre 0,001 et 0, 1 avantageusement avec un rapport égal à 0,05,
- avec de l'acide glyoxylique en solution aqueuse de concentration pondérale supérieure à 50%, avantageusement avec une solution aqueuse d'acide glyoxylique à 66% en poids.

La salification peut être réalisée selon les techniques bien connues de l'homme de l'art, par exemple à l'aide d'un hydroxyde alcalin, notamment l'hydroxyde de sodium.

La réaction de condensation de l'acide glyoxylique avec le phénol est avantageusement suivie par l'analyse chromatographique d'une prise d'essai prélevée régulièrement dans le milieu réactionnel. L'analyse chromatographique peut être, notamment, réalisée comme décrit par A.J Hoefnagel et al (loc.citée).

Lorsque la réaction est terminée, c'est à dire lorsque l'acide glyoxylique mis en oeuvre a été entièrement consommé, l'acide orthohydroxymandélique cherché est isolé du milieu réactionnel par des moyens connus en soi. Avantageusement, le milieu réactionnel refroidi est dilué avec une solution aqueuse d'hydroxyde de sodium, puis il est lavé avec un solvant organique non miscible à l'eau pour éliminer le phénol non transformé et l'amine tertiaire utilisée. La solution aqueuse résiduelle contenant l'acide orthohydroxymandélique salifié est ensuite soit utilisée telle quelle pour préparer l'acide orthohydroxyphénylacétique par exemple par réaction avec un agent donneur d'hydrogène tel que l'acide formique en présence d'un catalyseur de transfert d'hydrogène tel que le palladium sur charbon, de préférence dans un solvant tel que l'eau ou l'acide acétique, soit acidifiée à pH = 1,5 avec de l'acide chlorhydrique concentré, lavée ensuite avec de l'acétate d'éthyle pour extraire l'acide orthohydroxymandélique ; les phases organiques d'extraction sont ensuite concentrées sous pression réduite, puis l'huile résiduelle dissoute dans le minimum d'éthanol laisse déposer des cristaux d'orthohydroxymandélate de sodium pur lorsqu'on traite avec une solution d'hydroxyde de sodium dans de l'éthanol.

Les exemples 1-10 du procédé selon l'invention, ainsi que les exemples de comparaison C1-C11 ont été réalisés sous agitation, en atmosphère inerte, à la température $\Theta$ pendant le temps t (valeurs données dans le tableau I). Les essais réalisés au départ de glyoxylate de sodium, désigné AGNa, ont été effectués à un pH ajusté à 5 par addition si nécessaire d'une solution aqueuse concentrée d'hydroxyde de sodium. Le nombre de mmoles d'acide orthohydroxymandélique , désigné N, d'acide parahydroxymandélique, désigné M, et d'acide hydroxybenzènediglycolique-2,4 et -2,6, désigné P, ont été déterminés par chromatographie liquide à haute pression selon le protocole donné par A.J Hoefnagel (loc.citée), mais le pic identifié comme correspondant au produit disubstitué en 2,6 correspond en réalité au produit disubstitué en 2,4.

La sélectivité de la réaction, désignée S, est calculée selon l'équation 1, le taux de disubstitution, désigné T, est calculé selon l'équation 2, et le rendement R est calculé selon l'équation 3 dans laquelle L désigne le nombre de mmoles d'acide glyoxylique libre ou salifié mis en oeuvre. Dans les essais L est égal à 100.

$$S = \frac{100\ N}{M + N + 2P} \qquad\qquad \text{(equ 1)}$$

$$T = \frac{P\ 100}{M + N + 2P} \qquad\qquad \text{(equ 2)}$$

$$R = \frac{(M + N + 2P)\ 100}{L} \qquad\qquad \text{(equ3)}$$

L'exemple de comparaison C9 est la reproduction de l'exemple 1 du brevet FR 2638740 effectué en présence de sulfate d'aluminium.

Dans le tableau I, les abréviations utilisées ont la signification suivante :

| | |
|---|---|
| AG | : Acide glyoxylique exprimé en 100% |
| AGNa | : Glyoxylate de sodium exprimé en 100% |
| n | : Nature, soit du catalyseur, soit de l'amine utilisés |
| d | : Dose exprimée en mmoles |
| $\Theta$ | : Température exprimée en degrés Celsius |
| t | : Temps exprimé en minutes |
| S | : Sélectivité |
| T | : Taux de disubstitution |
| R | : Rendement calculé par rapport à l'acide glyoxylique mis en oeuvre |
| A | : Tributylamine |
| B | : Chlorure de chrome (III) cristallisé avec 6 moles d'eau PM = 266,45 |
| D | : Sulfate d'aluminium cristallisé avec 18 moles d'eau PM = 666,42 |
| F | : Chlorure de fer (III), PM = 162,21 |
| NM | : Non mesurable (voisin de 0) |
| $H_2O$ | : eau - lorsque le catalyseur utilisé contient de l'eau de cristallisation, cette eau n'est pas comprise dans les valeurs indiquées dans cette rubrique. |

L'examen du tableau I permet de tirer les conclusions suivantes :

- les essais effectués en milieu aqueux dilué ne permettent pas d'éviter la formation de produits disubstitués sur le phénol, même en présence d'un excès de phénol par rapport à l'acide glyoxylique (C1-C5).
- Les essais effectués avec un excès d'amine tertiaire par rapport à l'acide glyoxylique, et en présence d'un cation métallique trivalent ne permettent pas d'obtenir une sélectivité en para (C9).
- Les essais effectués avec le glyoxylate de sodium en présence d'un cation métallique trivalent et d'un excès de phénol, mais en milieu quasi anhydre ne permettent pas d'obtenir un taux de substitution significatif (C6).

# TABLEAU I

| | AG (mmoles) | AGNa (mmoles) | Phénol (mmoles) | $H_2O$ (mmoles) | Catalyseur nature | Catalyseur dose (mmoles) | Amine n | Amine d (mmole) | $\Theta$ (°C) | t (min) | S (%) | T (%) | R (%) |
|---|---|---|---|---|---|---|---|---|---|---|---|---|---|
| **EXEMPLES 1 - 10** | | | | | | | | | | | | | |
| 1 | 100 | 0 | 800 | 210 | D | 5 | A | 95 | 50 | 465 | 86,5 | NM | 100 |
| 2 | 100 | 0 | 800 | 210 | D | 5 | A | 95 | 80 | 120 | 86 | NM | 98 |
| 3 | 100 | 0 | 800 | 210 | D | 5 | A | 95 | 80 | 180 | 86 | NM | 98 |
| 4 | 100 | 0 | 800 | 210 | D | 5 | A | 95 | 100 | 30 | 87,5 | NM | 94 |
| 5 | 100 | 0 | 800 | 210 | D | 5 | A | 95 | 115 | 9 | 86,7 | NM | 91 |
| 6 | 100 | 0 | 800 | 210 | D | 5 | A | 95 | 115 | 19 | 86 | NM | 91 |
| 7 | 100 | 0 | 800 | 210 | F | 10 | A | 95 | 100 | 5 | 87 | NM | 92 |
| 8 | 100 | 0 | 800 | 210 | F | 2 | A | 95 | 100 | 30 | 84,5 | NM | 99 |
| 9 | 100 | 0 | 800 | 210 | F | 1 | A | 95 | 100 | 60 | 73,5 | NM | 99 |
| 10 | 100 | 0 | 800 | 210 | B | 1,25 | A | 95 | 100 | 120 | 59 | NM | 97 |
| **EXEMPLES DE COMPARAISON 1 - 11** | | | | | | | | | | | | | |
| C1 | 0 | 100 | 100 | 9840 | D | 5 | | 0 | 100 | 240 | 44 | 20 | 75 |
| C2 | 0 | 100 | 200 | 9840 | D | 5 | | 0 | 100 | 240 | 55 | 15 | 95 |
| C3 | 0 | 100 | 400 | 9840 | D | 5 | | 0 | 100 | 240 | 70 | 6,4 | 98 |
| C4 | 0 | 100 | 500 | 9840 | D | 5 | | 0 | 100 | 240 | 76 | 6,5 | 98 |
| C5 | 0 | 100 | 600 | 9840 | D | 5 | | 0 | 100 | 240 | 72 | 6,4 | 98 |
| C6 | 0 | 100 | 800 | 120 | D | 5 | | 0 | 100 | 240 | NM | NM | 2 |
| C7 | 0 | 100 | 200 | 9840 | D | 5 | | 0 | 100 | 360 | 69 | 10,7 | 87 |
| C8 | 0 | 100 | 200 | 9840 | D | 5 | | 0 | 80 | 1200 | 52,5 | 11,2 | 98 |
| C9 | 100 | 0 | 800 | 210 | D | 5 | A | 150 | 100 | 60 | 19 | NM | 97 |
| C10 | 0 | 100 | 500 | 9840 | F | 10 | | 0 | 100 | 90 | 49 | 7,4 | 94 |
| C11 | 0 | 100 | 500 | 9840 | B | 1,25 | | 0 | 100 | 240 | 76 | 3 | 91 |

4

**Revendications**

1. Procédé de préparation de l'acide orthohydroxymandélique, ainsi que de ses sels, caractérisé en ce que l'on fait réagir de l'acide glyoxylique avec du phénol en présence d'une amine tertiaire avec un rapport molaire amine tertiaire sur acide glyoxylique compris entre 0,8 et 1,2 et de quantités catalytiques de cations métalliques trivalents, pour obtenir le produit attendu que l'on isole et, si désiré, salifie.

2. Procédé selon la revendication 1, caractérisé par le fait que le rapport molaire amine tertiaire sur acide glyoxylique est compris entre 0,9 et 1,1.

3. Procédé selon la revendication 1 ou 2, caractérisé par le fait que l'amine tertiaire est liquide ou gazeuse à la température ambiante.

4. Procédé selon la revendication 3, caractérisé par le fait que l'amine tertiaire est de formule générale (I)

$$N(R_1)\,(R_2)\,(R_3) \tag{I}$$

dans laquelle $R_1$, $R_2$ et $R_3$, identiques ou différents présentent un radical alcoyle linéaire ou ramifié en $C_1$-$C_{10}$, ou un radical alcanol en $C_2$.

5. Procédé selon la revendication 4, caractérisé par le fait que l'amine de formule générale (I) est la tributylamine.

6. Procédé selon l'une quelconque des revendications 1 à 5, caractérisé par le fait que le cation métallique trivalent est choisi dans le groupe constitué par l'aluminium, le chrome, le fer.

7. Procédé selon la revendication 6, caractérisé par le fait que le cation métallique trivalent est le cation aluminium.

8. Procédé selon l'une quelconque des revendications 1 à 7, caractérisé par le fait qu'il est effectué à une température supérieure ou égale à 50°C.

9. Procédé de préparation de l'orthohydroxymandélate de sodium selon l'une des revendications 1 à 8, caractérisé par le fait qu'il est obtenu par salification de l'acide orthohydroxymandélique avec de l'hydroxyde de sodium.

**Claims**

1. Preparation process for orthohydroxymandelic acid, as well as its salts, characterized in that glyoxylic acid is reacted with phenol in the presence of a tertiary amine with a molar ratio of tertiary amine to glyoxylic acid comprised between 0.8 and 1.2 and catalytic quantities of trivalent metal cations, in order to obtain the expected product which is isolated and, if desired, salified.

2. Process according to claim 1, characterized by the fact that the molar ratio of tertiary amine to glyoxylic acid is comprised between 0.9 and 1.1.

3. Process according to claim 1 or 2, characterized by the fact that the tertiary amine is liquid or gaseous at ambient temperature.

4. Process according to claim 3, characterized by the fact that the tertiary amine is of general formula (I)

$$N(R_1)\,(R_2)\,(R_3) \tag{I}$$

in which $R_1$, $R_2$ and $R_3$, being identical or different represent a linear or branched $C_1$-$C_{10}$ alkyl radical or a $C_2$ alkanol radical.

5. Process according to claim 4, characterized by the fact that the amine of general formula (I) is tributylamine.

6. Process according to any one of claims 1 to 5, characterized by the fact that the trivalent metal cation is chosen from the group constituted by aluminium, chromium, iron.

7. Process according to claim 6, characterized by the fact that the trivalent metal cation is the aluminium cation.

8. Process according to any one of claims 1 to 7, characterized by the fact that it is carried out at a temperature greater than or equal to 50°C.

9. Preparation process for sodium orthohydroxymandelate according to one of claims 1 to 8, characterized by the fact that it is obtained by the salification of orthohydroxymandelic acid with sodium hydroxide.

**Patentansprüche**

1. Verfahren zur Herstellung von ortho-Hydroxymandelsäure sowie ihrer Salze, dadurch gekennzeichnet, daß man Glyoxylsäure mit Phenol in Gegenwart eines tertiären Amins mit einem molaren Verhältnis von tertiärem Amin zu Glyoxylsäure im Bereich von 0,8 bis 1,2 und katatlytischer Mengen dreiwertiger Metallkationen umsetzt, wobei das gewünschte Produkt erhalten wird, das isoliert und gegebenenfalls in ein Salz überführt wird.

2. Verfahren nach Anspruch 1, dadurch gekennzeichnet, daß das molare Verhältnis von tertiärem Amin zu Glyoxylsäure im Bereich von 0,9 bis 1,1 liegt.

3. Verfahren nach Anspruch 1 oder 2, dadurch gekennzeichnet, daß das tertiäre Amin bei Umgebungstemperatur flüssig oder gasförmig ist.

4. Verfahren nach Anspruch 3, dadurch gekennzeichnet, daß das tertiäre Amin die allgemeine Formel (I) aufweist,

$$N(R_1)\ (R_2)\ (R_3) \tag{I}$$

, in der $R_1$, $R_2$ und $R_3$, die gleichartig oder verschieden sein können, einen linearen oder verzweigten $C_1$-$C_{10}$-Alkylrest oder einen $C_2$-Alkanolrest verkörpern.

5. Verfahren nach Anspruch 4, dadurch gekennzeichnet, daß das Amin der allgemeinen Formel (I) Tributylamin ist.

6. Verfahren nach einem der Ansprüche 1 bis 5, dadurch gekennzeichnet, daß das dreiwertige Metallkation aus der aus Aluminium, Chrom und Eisen bestehenden Gruppe ausgewählt ist.

7. Verfahren nach Anspruch 6, dadurch gekennzeichnet, daß das dreiwertige Metallkation das Aluminiumkation ist.

8. Verfahren nach einem der Ansprüche 1 bis 7, dadurch gekennzeichnet, daß es bei einer Temperatur von mehr als oder gleich 50°C durchgeführt wird.

9. Verfahren zur Herstellung von Natrium-orthohydroxymandelat nach einem der Ansprüche 1 bis 8, dadurch gekennzeichnet, daß es durch Salzbildung der ortho-Hydroxymandelsäure mit Natriumhydroxid erhalten wird.